## Europäisches Patentamt

## European Patent Office

## Office européen des brevets

(11) Veröffentlichungsnummer: **0 012 146**
**A1**

(12) **EUROPÄISCHE PATENTANMELDUNG**

(21) Anmeldenummer: **78810025.3**

(22) Anmeldetag: **23.11.78**

(51) Int. Cl.³: **A 61 F 1/00**

(43) Veröffentlichungstag der Anmeldung: **25.06.80**
**Patentblatt 80/13**

(84) Benannte Vertragsstaaten: **BE CH DE FR GB LU NL SE**

(71) Anmelder: **OSTEO AG, Bielstrasse 620, CH-2545 Selzach (CH)**

(72) Erfinder: **Mittelmeier, Heinz, Prof. Dr. med., An der Schutzhütte, Blieskastel (DE)**
Erfinder: **Moser, Heinz, Späretweg 440, CH-2545 Selzach (CH)**
Erfinder: **Leu, Beat, Hintere Gasse 33, CH-2500 Biel (CH)**

(74) Vertreter: **Seehof, Michel et al, c/o AMMANN PATENTANWAELTE AG BERN Schwarztorstrasse 31, CH-3001 Bern (CH)**

(54) **Zementfrei implantierbare, mit Tragrippen versehene Prothese zum Ersatz eines Gelenkes und Verfahren zur Herstellung dieser Prothesen.**

㊗ Bei der zementfrei implantierbaren, mit Tragrippen versehenen Prothese zum Ersatz eines Gelenkes bestehen die im Knochen zu verankernden Teile aus Kohlefaserverstärktem (17), im Körpermilieu mechanisch und chemisch stabilen Kunststoff (16), während die mit dem Knochen in Berührung stehenden Aussenflächen mit die Verankerung im Knochen fördernden Materialien in Partikelform (18) versehen sind. Die Partikel können aus einem Glaskeramikwerkstoff oder Kalziumphosphaten oder aus den mineralischen Komponenten des natürlichen Knochengewebes, Apatit, bestehen.

EP 0 012 146 A1

BEZEICHNUNG GEÄNDERT
siehe Titelseite

- 1 -

## Zementfrei implantierbare, mit Tragrippen versehene Prothese zum Ersatz eines Gelenkes

Die vorliegende Erfindung bezieht sich auf eine zementfrei implantierbare, mit Tragrippen versehene Prothese zum Ersatz eines Gelenkes, wobei in erster Linie an das Hüftgelenk gedacht ist, das heisst Gelenke, die einer hohen spezifischen Belastung ausgesetzt sind.

Eine zementfrei implantierbare, mit Tragrippen versehene Hüftgelenkprothese ist aus der DE-OS 24 11 617 bekannt, wobei in der auf dem Markt erhältlichen und in vielen Kliniken angewandten Ausführung die Hüftgelenkpfanne und der Kugelkopf aus Keramik gefertigt sind, während der Prothesenschaft aus einer Kobalt-Chrom-Molybdänlegierung besteht. Obwohl diese Prothese erfolgreich angewendet wird, besteht das Bestreben, einerseits die Verankerung noch zu verbessern, und andererseits den Preis einer solchen Prothese herabzusetzen.

Um das Anwachsen des Knochens an Prothesen zu verbessern, wurde bereits vorgeschlagen, an den Aussenflächen der Prothese eine geschlossene Schicht sogenannter bioaktiver Keramikwerkstoffe aufzutragen. Bei der Verwendung einer in sich zusammenhängenden Keramikschicht mit hohem E-Modul und relativ geringer Festigkeit ist jedoch, insbesondere bei einem Prothesenschaft mit hoher Biegebelastung, die Gefahr vorhanden, dass die Keramikschicht brechen und abbröckeln kann. Ausserdem ist poröse Keramik vorgeschlagen worden, welche durch Einwachsen von Knochengewebe in die Poren eine bessere Verankerung ermöglichen soll. Hier ist jedoch die Gefahr des Keramik-Bruches infolge der Porosierung in erhöhtem Mass gegeben.

Ausserdem bestehen bei Prothesen aus keramischem Werkstoff infolge des, im Vergleich zum Knochen, hohen E-Moduls Relativbewegungen in der Grenzschicht, welche bei Belastung eine unmittelbare Verbindung zwischen der Keramik und dem Knochengewebe trotz der bestehenden physikalisch chemischen Verbindungskräfte verhindern können. Zur Verhütung von störenden Relativbewegungen an der Grenzschicht, infolge unterschiedlicher Elastizitätsverhältnisse von Prothese und Knochen, wurde die sogenannte "Isoelastische Prothese", beschrieben in "Total Hip Prosthesis", Professor Gschwend and Professor Debrunner, H. Huber Verlag, Bern 1976, aus einem Werkstoff mit gleichem E-Modul wie der Knochen geschaffen. Hierbei treten jedoch gleichfalls Relativbewegungen in der Grenzschicht auf, wenn dabei nicht eine den regionalen Knochenverhältnissen entsprechende Steifigkeit des Prothesenkörpers erreicht wird.

Aus der DE-OS 26 20 907 ist eine Metallprothese bekannt, deren Schaft mit einer etwa 2 - 3 mm dicken Schicht aus polymeren Kunststoffen mit darin eingebetteten Kugeln aus resorbierbarer Kalziumphosphatkeramik versehen ist. Einerseits weist diese Prothese keine Tragrippen auf, so dass die Vergrösserung der tragenden Fläche durch die Keramikpartikel allein gering ist und erst nach deren Resorption porotische Höhlen entstehen, in die der Knochen unter Oberflächenvergrösserung einwachsen kann. Ausserdem besteht die Gefahr einer Zerreissung der porosierten Kunststoffschicht bzw. Abscherung vom zentralen Metallschaft.

Gleiches gilt für eine primär porosierte Kunststoffbeschichtung (ohne Einlagerung von resorbierbaren Kalziumphosphatkeramikkugeln) gemäss US-PS 1 505 215.

Aus der US-PS 4 055 862 ist eine Knieprothese bekannt, die aus kohlefaserverstärktem Polyäthylen besteht, jedoch keine Tragrippen aufweist und daher einzementiert werden muss.

Es ist demgegenüber Aufgabe der vorliegenden Erfindung, eine zementfrei implantierbare Prothese anzugeben, deren Steifigkeit und Oberflächenelastizität den regionalen Verhältnissen entspricht und die bessere primäre mechanische Verankerung aufweist.

Dieses Ziel wird dadurch erreicht, dass die im Knochen zu verankernden Teile aus faserverstärktem, im Körper mechanisch und chemisch stabilem Kunststoff bestehen und dass sie an ihren mit dem Knochen in Berührung stehenden Aussenflächen mit die Verankerung im Knochen fördernden und die tragende Oberfläche vergrössernden Materialien in Partikelform versehen sind. Bevorzugte Ausführungsbeispiele weisen Partikel aus einem Glaskeramikwerkstoff und/oder aus den mineralischen Komponenten des natürlichen Knochengewebes, Apatit, auf, wobei die Fasern Kohlefasern sind.

Die Erfindung wird nun anhand einer Zeichnung eines Ausführungsbeispiels näher erläutert werden, wobei

Figur 1 eine Hüftgelenkprothese,

Figur 2 einen Schnitt durch den Prothesenschaft gemäss Linie II-II und

Figur 3 einen schematisch und vergrössert dargestellten Ausschnitt aus einem Schnitt durch die Aussenoberfläche zeigt.

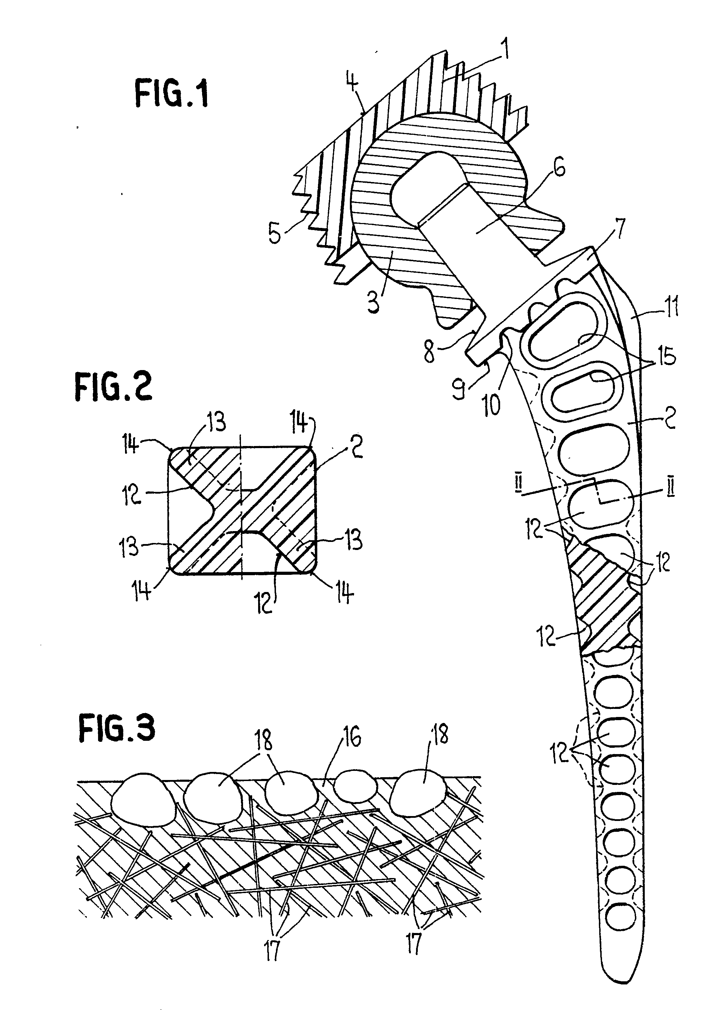

Bei Figur 1 ist eine Hüftgelenkprothese dargestellt, und man erkennt die Hüftgelenkpfanne 1, den Prothesenschaft 2 und den Kugelkopf aus Keramik 3.

Die Aussenfläche der Hüftgelenkpfanne 1 weist eine konische, sich zur Stirnseite 4 hin verjüngende Form auf, wobei die konische Aussenfläche ein als Tragrippen dienendes Gewinde 5 besitzt. Bekannterweise besitzt die Aussenfläche ferner Nuten zur Erhöhung der Rotationsstabilität und die untere, dem Prothesenschaft zugewandte Stirnseite Nuten zum Ansetzen des Eindrehinstrumentes.

Der Prothesenschaft 2 weist einen konischen Zapfen 6 zur rotationsstabilen Fixierung des Kugelkopfes, ein Plateau 7, dessen Oberseite 8 angeschrägt ist, um mittels eines Werkzeuges den Kopf vom Zapfen lösen zu können und dessen Plateauunterseite 9 Nuten 10 aufweist, die der Verbesserung der Rotationsstabilität dienen, auf. An der Lateralseite des Schaftes ist zwecks weiterer Erhöhung der Rotationsstabilität ein Flügel 11 angebracht. Der Umfang des Schaftes ist, wie aus Figur 2 hervorgeht, im wesentlichen rechteckig und weist an seinen vier Seiten Tragrippen 12 in Form von Einbuchtungen und Längsrippen 13 auf, wobei hervorzuheben ist, dass die Kanten 14 der Längsrippen 13 abgerundet sind, um Einkerbungen des anwachsenden Knochens zu vermeiden. Im oberen Teil des Schaftes sind zwei Fenster 15 zu Einbringen von Spongiosaspänen angebracht. Die Tragrippen bzw. Einbuchtungen und Längsrippen ergeben zusammen, wie dies aus Figur 2 hervorgeht, ein Doppel-Y-Profil des Schaftes. Wie ferner aus den Figuren 1 und 2 hervorgeht, sind die Tragrippen der längeren Seiten versetzt zu denjenigen der schmäleren Seiten angeordnet, derart, dass eine Tragrippe an der Schmalseite jeweils zwischen zwei Tragrippen der längeren Seite zu liegen kommt. Der konische Zapfen befindet sich in Valgusposition, wobei der Winkel zwischen dem konischen Zapfen und dem Schaft 140$^{\circ}$ beträgt, wodurch eine günstige Kraftverteilung gewährleistet wird.

Der Prothesenschaft und die Hüftgelenkpfanne besteht, wie in Figur 3 schematisch angedeutet, aus einem Kunststoff 16, vorzugsweise Polyäthylen, in welchem Kohlefasern 17 eingelagert sind.

Vorzugsweise sind die Kohlefasern nicht unregelmässig in den Kunststoff eingelagert, sondern in konstruktiver Weise den Belastungsspannungen entsprechend eingelagert, um hier einen relativ hohen E-Modul und Biegesteifigkeit zu erhalten, in der knochennahen Schicht jedoch soweit reduziert, dass der E-Modul annähernd den Knochenverhältnissen entspricht oder darunter liegt, damit in dieser Schicht Relativbewegungen zwischen Prothesenschicht und Knochenlager ausgeglichen werden können. In den hauptsächlich belasteten Zonen sollen dementsprechend nicht nur Kurzfasern, sondern lange Faserbündel, Fasergeflechte oder Fasernetze eingelagert werden. Dies gilt insbesondere für die randständigen längsgerichteten Stützpfeiler, bzw. Tragrippen der Verankerungsstiele, wie auch modulare Oberflächenkomponenten, z. B. die Pfannenschale der Hüftgelenke oder die Condylenüberzüge anderer Gelenke, beispielsweise bei Kniegelenkprothesen.

Dabei sollte Sorge getragen werden, dass die Fasern derart eingelagert werden, dass sie nicht in direkten Kontakt mit dem Knochen gelangen können.

Die Verankerungsteile der Prothese sollen eine grobe Profilierung aufweisen, welche eine mechanische Verankerung im Knochen gewährleisten, sei es, dass das Knochengewebe primär z. B. durch Schneiden eines Gewindes dem Oberflächenprofil der Prothese sofort angepasst wird und bei Verwendung des Schraubenprofils eine primäre Vorspannung ermöglicht; sei es, dass die Oberflächenvergrösserung des tragenden Knochengewebes zum

0012146

Zwecke der Reduzierung des Belastungsdruckes sekundär durch Einwachsen des Knochengewebes in die Profilbuchten der Prothese erfolgt, ein Prinzip, das aus der DE-OS 24 11 617 bereits für keramischen und metallischen Werkstoff hervorgeht.

Bei Verankerung in geraden Knochenschäften ist vorzugsweise an ein konisches unterbrochenes Aussengewinde auf dem Verankerungsstiel gedacht, welches den Vorteil eines Nachschneideeffektes (nach vorausgehendem Gewindeschneiden), zugleich auch einer Rückdrehsicherung, hat, im Unterschied zu einem nicht unterbrochenen Gewinde, das zusätzlicher Rückdrehsicherung bedarf. Es bleibt vorbehalten, die Längen der verbleibenden Gewindeteile und der Unterbrechungen zu variieren, eventuell auch derart, dass das Gewinde nur aus kurzen Noppen besteht (Noppengewinde). Dabei kann der Verankerungsstiel im Sinne der Materialeinsparung hohl gestaltet und das Stielende ein- oder mehrfach geschlitzt sein.

Sofern die unterfütternde Knochenpartie des Gelenkes keine ausreichende Tiefe besitzt, um einen langen Verankerungskonus einzuschrauben, beispielsweise im Bereich der Hüftpfanne, kann der konische Schraubkegel auch stumpfkegelig gestaltet sein.

Im Bereich gekrümmter Knochenmarkhöhlen, beispielsweise am prox. Femurende, wo ein Verankerungsstiel mit konischem Gewinde nicht ohne weiteres eingeschraubt werden kann, soll die Oberflächenprofilierung zirkuläre und longitudinale, eventuell auch schräge Tragrippen aufweisen, die auch wellenförmig oder durch zusätzliche Längsrippen wabenförmig gestaltet sind.

Ausser dieser groben Oberflächenprofilierung kann noch eine Feinprofilierung in Wabenform, vornehmlich in den

0012146

Tragrippenbuchten, vorgenommen werden, welche das Einwachsen von Knochengewebe und damit eine weitere Oberflächenvergrösserung und somit Druckreduktion beinhaltet.

Die optimale Prothesenverankerung wird wesentlich dadurch verbessert, dass die mit den Knochen in Berührung stehenden Aussenflächen der Prothesenteile mit kleinen Partikeln bioaktiven Materials bestückt sind, welche ein unmittelbares Anwachsen des Knochens mit zusätzlicher physikalisch/chemischer Bindung ermöglicht. Als bioaktives Material sollen - im wesentlichen Unterschied zur DE-OS 26 20 907 - nicht resorbierbare Stoffe dienen, insbesondere Glaskeramik, bei der Apatit, das natürliche Knochenmaterial, in einer Silikatmatrix gebunden ist. Ersatzweise könnten auch feine Partikelchen aus enteiweisstem und entfettetem Knochengewebe Verwendung finden, wobei aus Immunitätsgründen auto- und homoplastischem Material vor heteroplastischem der Vorzug zu geben wäre.

Die bioaktiven Partikel 18 können beim Fertigungsprozess so in die oberflächliche Kunststoffschicht eingepresst werden, dass sie vom Kunststoff überäquatorial aber unvollständig umklammert werden, ähnlich wie Edelsteine in einer Schmuckfassung. Auf diese Weise haben sie noch eine ausreichende Oberfläche gegen den Knochen, sind jedoch andererseits durch die Kunststoffassung wie durch Dehnungsfugen voneinander getrennt. Im Unterschied zur Flächenbeschichtung ist damit weder ein Bruch noch ein Abblättern der bioaktiven Keramikschicht zu befürchten. Andererseits bewirken die vorstehenden Partikel eine weitere mikroskopische Oberflächenvergrösserung und Verzahnung. Es kann auch zweckmässig sein,verschiedene Materialien, d. h. Apatit, Kalziumphosphate und andere keramische oder glaskeramische Werkstoffe gleichzeitig zu verwenden und für verschiedene Stellen, je nach Art der Belastung und Eigenschaften des umgebenden Knochengewebes, verschiedene Partikel unterschiedlicher Grösse und aus verschiedenen Materialien vorzusehen.

Die Kohlefasern können entweder, wie in Figur 3 angedeutet, durcheinander liegen oder, insbesondere an bestimmten Stellen, wie beispielsweise an der Oberfläche des konischen Zapfens oder an der Gelenkfläche der Pfanne, eine bestimmte bevorzugte Ausrichtung aufweisen, wobei sowohl die Länge als auch die Dicke der Fasern verschiedene, dem Problem angepasste Abmessungen haben können. durch die Auswahl eines geeigneten Kunststoffes wie beispielsweise Polyäthylen, und eines bestimmten Prozentsatzes von Kohlefasern ist es möglich, einen Verbundwerkstoff herzustellen, der den hohen Anforderungen von Prothesen entspricht, wobei die in Frage kommenden Parameter je nach Art der Prothese variiert werden können.

Verfahren zur Herstellung von faserverstärkten Kunststoffteilen sind bekannt, wobei in der Regel solche Teile in einem Arbeitsgang geformt werden. Zwecks Verminderung der Reibung der Gelenkflächen kann es zweckmässig sein, die Gelenkflächen der Pfanne in einem weiteren Arbeitsgang nachzubearbeiten, beispielsweise durch Hineinpressen einer dem Kugelkopf entsprechenden Keramikkugel im warmen Zustand.

Um vorgehend beschriebene Prothesenteile herzustellen ist es zweckmässig, die Form an den gewünschten, mit dem Knochen in Berührung kommenden Stellen mit einer geeigneten Klebelösung zu bestreichen und die oben angegebenen Partikel in vorgegebener Menge darüberzustreuen, wobei sie nur an den mit der Lösung bestrichenen Stellen haften bleiben. Hierauf wird der mit den in geeigneter Anordnung geschichteten und eventuell ausgerichteten Kohlefasern versehene Kunststoff in die Form eingebracht. Anschliessend wird das Prothesenteil nachbearbeitet, wobei Klebelösungsreste und nicht fest

0012146

haftendende Partikelteile entfernt und gegebenenfalls die Gelenkflächen, wie oben erwähnt, nachbearbeitet werden.

Alternativ zu dem oben angegebenen Verfahren ist es möglich, zuerst das Prothesenteil mit dem faserverstärkten Kunststoff zu formen und die Partikel entweder in einer geeigneten Pressform oder in die bereits der Herstellung des Prothesenteiles dienende Form mittels der Klebelösung anzubringen, das Prothesenteil einzulegen und die Formteile zu schliessen und zu erhitzen.

Bei der Wahl einer geeigneten Temperatur dringen die Partikel teilweise in die erweichte Oberfläche des Prothesenteils ein und werden dort verankert.

Dieses Verfahren ist vor allem geeignet, abgegrenzte Gebiete zu beschichten oder auf bestimmten Oberflächen bestimmte Partikel aufzubringen, wobei für das Bestreuen der Formen Abdeckmasken verwendet werden können.

Wie bereits eingangs erwähnt wurde, sind der faserverstärkte Kunststoff und das beschriebene Beschichtungsverfahren nicht nur für die Herstellung von Hüftgelenkprothesen geeignet, sondern für die Herstellung aller Arten von Prothesen, insbesondere auch solche, die zum Ersatz von Knie- und Schultergelenken gedacht sind, d. h. insbesondere von solchen Gelenken, die einer hohen spezifischen Belastung ausgesetzt sind. Durch die Verwendung von Kohlefasern und der Beschichtung durch verhältnismässig harte Partikel ist es möglich, die bereits bewährten Operationstechniken für Keramik- oder Metallprothesen beizubehalten, wobei es insbesondere möglich ist, die Hüftgelenkpfanne einzudrehen.

Hinsichtlich der artikulierenden Gelenkteile selbst, also Pfanne und Kopf bzw. Gelenkrolle, besteht die Möglichkeit, die beiden Prothesenteile unmittelbar als Pfanne und Kopf bzw. Gelenkrolle auszugestalten. Im Hinblick auf die Verbesserung der Reibungsverhältnisse erscheint jedoch die Bestückung der artikulierenden Gelenkteile mit reibungsgünstigen Materialien wünschenswert. Vorzugsweise sollen die konkaven Gelenkteile aus dem faserverstärkten Kunststoff und die konvexen Teile aus $AL_2O_3$-Keramik gefertigt werden. Hier liegen besonders günstige Verhältnisse mit geringfügigem Abrieb und kleinem Reibungskoeffizenten vor. Obwohl vorzugsweise an eine Kohlefaserverstärkung gedacht ist, können auch andere geeignete Materialien wie Bor oder andere Metallfasern verwendet werden, die jedoch nicht mit dem Knochengewebe in Berührung kommen dürfen.

0012146

- 1 -

Patentansprüche:

1. Zementfrei implantierbare, mit Tragrippen versehene Prothese zum Ersatz eines Gelenkes,
dadurch gekennzeichnet,
dass die im Knochen zu verankernden Teile (1, 2) aus faserverstärktem, im Körpermilieu mechanisch und chemisch stabilem Kunststoff (16) bestehen und dass sie an ihren mit dem Knochen in Berührung stehenden Aussenflächen mit die Verankerung im Knochen fördernden und die tragende Oberfläche vergrössernden Materialien in Partikelform (18) versehen sind.

2. Prothese gemäss Anspruch 1,
dadurch gekennzeichnet,
dass die Fasern Kohlefasern (17) in Form von Kurzfasern, Faserbündeln, Fasergeflechte oder Fasernetze sind.

3. Prothese gemäss Anspruch 2,
dadurch gekennzeichnet,
dass die Kohlefasern entsprechend den Belastungsspannungen angeordnet sind, wobei in knochennahen Schichten die Anzahl Fasern reduziert ist, derart, dass der E-Modul demjenigen des Knochens nahekommt.

4. Prothese gemäss Anspruch 2 oder 3,
dadurch gekennzeichnet, dass die Kohlefasern in Bezug auf die Prothesenoberfläche ausgerichtet sind.

5. Prothese gemäss Anspruch 1,
dadurch gekennzeichnet,
dass die nicht resorbierbaren Partikel aus bioaktiver
Glaskeramik bzw. aus der in einer Silikatmatrix eingebetteten mineralischen Komponente des natürlichen
Knochengewebes, Apatit, und/oder aus enteiweisstem und
entfetteten Knochengewebe bestehen.

6. Prothese gemäss einem der Ansprüche 1 - 5, zum Ersatz
eines Hüftgelenkes,
dadurch gekennzeichnet,
dass die Hüftgelenkpfanne (1) eine äussere konische
sich zur Stirnfläche (4) hin verjüngende Form aufweist
und die Tragrippen (5) als unterbrochenes, rückdrehsicheres Gewindeprofil ausgebildet sind.

7. Prothese gemäss einem der Ansprüche 1 - 6 zum Ersatz
eines Hüftgelenkes,
dadurch gekennzeichnet,
dass der Prothesenschaft (2) ein Doppel-Y-Profil aufweist und dass die Tragrippen (12) als Vertiefungen
ausgebildet und die sich gegenüberliegenden Vertiefungen
jeweils paarweise versetzt angeordnet sind und insbesondere in den Vertiefungen eine wabenförmige Feinprofilierung aufweisen.

8. Prothese gemäss Anspruch 6 oder 7,
dadurch gekennzeichnet,
dass der Kugelkopf (3) aus Aluminiumoxydkeramik gefertigt ist.

9. Verfahren zur Herstellung von Prothesen gemäss den Ansprüchen 1 - 8,

0012146

dadurch gekennzeichnet,

dass die Teile der Pressform, die den zu beschichtenden Stellen des Prothesenteiles entsprechen, mit einem
Haftmittel und mit den gewünschten Partikeln versehen werden, woraufhin der Kunststoff mit den in geeigneter Anordnung geschichteten und gegebenenfalls
bezüglich der Oberfläche ausgerichteten Kohlefasern in
die Pressform eingebracht wird.

10. Verfahren zur Herstellung von Prothesen gemäss den
Ansprüchen 1 - 8,
dadurch gekennzeichnet,
dass zuerst der Kunststoff zu einem Prothesenteil mit
geordneten Kohlefasern geformt wird und anschliessend
diejenigen Teile der Pressform, die den zu beschichtenden Stellen des Prothesenteiles entsprechen, mit einem
Haftmittel und mit den gewünschten Partikeln versehen
werden, woraufhin die Partikel unter Erwärmung auf das
Prothesenteil gepresst werden, um dort teilweise einzudringen.

11. Verfahren gemäss Anspruch 9 oder 10,
dadurch gekennzeichnet,
dass insbesondere die Gelenkflächen einer Nachbearbeitung
unterzogen werden.

FIG.1

FIG.2

FIG.3

# EUROPÄISCHER RECHERCHENBERICHT

Europäisches Patentamt

Nummer der Anmeldung

EP 78 81 0025

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 339 388 (BOSCH)  * Seite 2, Zeile 26 bis Seite 5, Zeile 27 * | 1,2,3, 4,10 |
| | -- | |
| | FR - A - 2 104 009 (HOCHMAN)  * Seite 2, Zeile 34 bis Seite 4, Zeile 23 * | 1,2,3, 4,10, 11 |
| | -- | |
| | FR - A - 2 361 093 (AUGSBURG-NÜRNBERG) | 1,2,3, 4 |
| | * Seite 2, Zeile 13 bis Seite 4, Zeile 37 * | 1,2,3, 4 |
| | -- | |
| | FR - A - 2 327 758 (WETZLAR)  * Seite 2, Zeile 16 bis Seite 7, Zeile 21 * | 1,5,11 |
| | -- | |
| | FR - A - 2 350 824 (PFAUDLER)  * Seite 8, Zeile 18 bis Seite 10; Figur 7 * | 1,5 |
| | -- | |
| | DE - A - 2 645 100 (HOFMEISTER)  * Seite 8, Zeilen 16 bis 25; Figur 3 * | 6,7 |
| | -- | |
| | DE - A - 2 628 284 (CERAVER)  * Seite 3, Zeile 8 bis Seite 4, Zeile 16; Figur 2 * | 7 |
| | -- | |
| | DE - A - 2 451 275 (DORRE)  * Seite 3, Zeilen 1 bis 12 * | 8 |
| | -------- | |

**KLASSIFIKATION DER ANMELDUNG (Int.Cl.²)**

A 61 F 1/00

**RECHERCHIERTE SACHGEBIETE (Int. Cl.²)**

A 61 F

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 20-07-1979 | LEMERCIER |

EPA form 1503.1  06.78